# EUROPEAN PATENT APPLICATION

(11) **EP 4 104 839 A1**
(43) Date of publication of application: **21.12.2022**
(21) Application number: 20919001.6
(22) Date of filing: 10.09.2020
(51) Int. Cl.: A61K 31/565, A61K 31/375, A61P 19/10, A61P 15/00

(54) **17BETA-ESTRADIOL AND VITAMIN C MOLECULAR COMPLEX, PREPARATION METHOD THEREFOR AND APPLICATION THEREOF**

(30) Priority: 12.02.2020 CN 202010087899
(71) Applicant: ZheJiang Medicine Co., Ltd. Xinchang Pharmaceutical Factory, Xinchang, Zhejiang 312500 (CN)
(72) Inventor: PENG, Shiqi, Xinchang Zhejiang 312500 (CN); WU, Guofeng, Xinchang Zhejiang 312500 (CN); ZHAO, Ming, Xinchang Zhejiang 312500 (CN)
(74) Representative: Balder IP Law, S.L.
(86) International application number: PCT/CN2020/114551
(87) International publication number: WO 2021/159704

(57) **Abstract**

The present disclosure discloses a 17β-estradiol/vitamin C molecular complex, and a preparation method and a use thereof. The molecular complex is obtained by compounding of 17β-estradiol and vitamin C in a molar ratio of 0.25:1, 0.5:1, 0.75:1, 1:1, 1:0.25, 1:0.5, or 1:0.75. The present disclosure also provides a preparation method of the 17β-estradiol/vitamin C molecular complex. The 17β-estradiol/vitamin C molecular complex can be distributed to a bone tissue in a bone-targeted manner, which not only significantly improves the anti-osteoporosis activity of 17β-estradiol but also effectively prevents the side effects of endometrial hyperplasia and thrombosis caused by treatment with 17β-estradiol and conjugated estrogens.

## Description

### TECHNICAL FIELD

The present disclosure relates to a 17β-estradiol/vitamin C molecular complex, and in particular, to a 17β-estradiol/vitamin C molecular complex obtained by compounding 17β-estradiol with vitamin C in a molar ratio of 1:1 and a preparation method thereof. The present disclosure further relates to a use of the molecular complex in the preparation of an anti-osteoporosis drug. The present disclosure belongs to the technical field of medicine.

### BACKGROUND

17β-estradiol (E2) is involved in the physiological and pathological activities of animals and humans. For example, 17β-estradiol can increase the survival and activation of new neurons in the hippocampi of adult female rats that respond to the spatial memory, improve the cognitive functions of humans and animals, and attenuate the oophorectomy-induced changes in the myocardial contraction function. For another example, mice treated by 17β-estradiol can be administered with melatonin to inhibit the expression of ovarian aromatase, thereby increasing the expression of uterine receptors. The 17β-estradiol-dependent production of reactive oxygen species (ROS) in vascular smooth muscle cells (VSMCs) is mediated in part by the induction of 12-hydroxyeicosatetraenoic acid (12-HETE) and 15-hydroxyeicosatetraenoic acid (15-HETE). 17β-estradiol can improve the bone healing in ovariectomized rats with skull defects who receive bovine bone grafting. The loss of ovarian hormones (especially 17β-estradiol) during natural or surgical menopause may lead to a series of symptoms that can compromise the quality of life. 17β-estradiol can be particularly used for hormone replacement therapy (HRT) in postmenopausal women to improve health problems triggered by 17β-estradiol deficiency. Such health problems include decreased communicative interaction ability, biased mate-selection preference and behavior, working memory fatigue, behavioral changes, operant deficit task in delayed spatial transformation assignment, increased bone turnover, and decreased mitochondrial function.

Menopause is a physiological phenomenon caused by the loss of ovarian function. The loss of ovarian function during menopause can lead to a decrease in concentrations of ovarian hormones in the body, such as estrogen and progestin. Although the HRT targeting 17β-estradiol supplementation can protect the bone health in postmenopausal women, the Women's Health Study (WHS) has reported that the HRT may cause adverse reactions, such as uterine weight gain and thromboembolism. Such adverse reactions call into question the HRT targeting 17β-estradiol supplementation. However, recent studies suggest that the adverse reactions depend on the timing and duration of treatment. As clinical medical and epidemiological data further highlight the need for the HRT targeting 17β-estradiol supplementation, researchers have made much effort to improve the efficacy and safety of 17β-estradiol therapy. New progestins are used in a number of studies, for example, representative 17β-estradiol and dydrogesterone combined therapy, plant-derived 17β-estradiol analogues, 17β-estradiol-loaded electrospun polyurethane-dextran nanofiber mats, 17β-estradiol-loaded poly(DL-lactide-co-glycolide) nanoparticles, 17β-estradiol-loaded PEGylated upconverting nanoparticles, and polyesteramide (PEA) for bone regeneration and controlled release are used. However, the research suggests that the adverse reactions caused by 17β-estradiol therapy may not be overcome, and even conjugated estrogens still lead to the adverse reactions of 17β-estradiol.

It is an ongoing interest of the inventors of the present disclosure to study complexes of 17β-estradiol to overcome the adverse reactions. After 10 years of exploration, the inventors discover that a 17β-estradiol/vitamin C molecular complex can not only improve the anti-osteoporosis activity of 17β-estradiol but also eliminate the two lethal side effects of endometrial hyperplasia and thrombosis caused by 17β-estradiol. Based on these findings, the present disclosure is proposed.

### SUMMARY

In order to overcome the adverse reactions of 17β-estradiol therapy in the prior art, the inventors unexpectedly discovered through 10 years of exploration that a 17β-estradiol/vitamin C molecular complex can be distributed to a bone tissue in a bone-targeted manner, which not only improves the anti-osteoporosis activity of 17β-estradiol but also eliminates the two lethal side effects of endometrial hyperplasia and thrombosis caused by 17β-estradiol.

In order to achieve the above objective, the present disclosure adopts the following technical solutions:
The present disclosure provides a 17β-estradiol/vitamin C molecular complex, where the molecular complex is obtained by compounding 17β-estradiol with vitamin C, in which a distance between an aromatic proton in the 17β-estradiol and an enol proton in the vitamin C is less than 4 Å.

Preferably, the molecular complex may be obtained by compounding the 17β-estradiol with vitamin C in a molar ratio of 0.25:1, 0.5:1, 0.75:1, 1:1, 1:0.25, 1:0.5, or 1:0.75.

Preferably, the molecular complex may be obtained by compounding the 17β-estradiol with vitamin C in a molar ratio of 1:1.

Preferably, the mass number of the molecular ion of the molecular complex in an ESI(+)-FT-MS spectrum may be 449.21420, which is equal to a mass number of one 17β-estradiol molecule: 272.17763 + a mass number of one vitamin C molecule: 176.03209 + 1 H.

Preferably, in a qCID spectrum of the molecular complex under ESI(+)-FT-MS conditions, the molecular ion with a mass number of 449.21420 may be split into a 17β-estradiol ion with a mass number of 273.18455 + 1 H and a vitamin C ion with a mass number of 199.02143 + 1 Na, that is, a 17β-estradiol/vitamin C molecular complex in a molar ratio of 1:1 is the only form in which 17β-estradiol and vitamin C exist.

Further, the present disclosure also provides a preparation method of the 17β-estradiol/vitamin C molecular complex, including the following steps:
1) mixing 17β-estradiol and vitamin C in a molar ratio of 0.25:1, 0.5:1, 0.75:1, 1:1, 1:0.25, 1:0.5, or 1:0.75 to obtain a mixture;
2) dissolving the mixture in an aqueous ethanol solution to prepare a clear solution; and
3) subjecting the clear solution to solvent removal to obtain a solid powder, which is the 17β-estradiol/vitamin C molecular complex.

Preferably, in step 1), a molar ratio of 17β-estradiol to vitamin C may be 1:1.

Preferably, in step 2), based on V/V, the aqueous ethanol solution may be a 30% to 60% aqueous ethanol solution.

Preferably, in step 3), the solvent may be removed by performing lyophilization, vacuum distillation, or spray drying; and more preferably, the solvent may be removed by performing lyophilization.

Furthermore, the present disclosure also provides a use of the 17β-estradiol/vitamin C molecular complex in the preparation of an anti-osteoporosis drug; and a use of the 17β-estradiol/vitamin C molecular complex in the preparation of a drug for inhibiting endometrial hyperplasia.

In the present disclosure, the anti-osteoporosis activities of the 17β-estradiol, conjugated 17β-estradiol, 17β-estradiol/vitamin C molecular complex are evaluated on an ovariectomized female mouse model. According to the trabecular bone density, the femoral dry weight, the femoral ash weight, the femoral mineral content, the femoral calcium content, and the ratio of the femoral ash weight to the femoral length, it was found that the 17β-estradiol/vitamin C molecular complex has prominent anti-osteoporosis activity. Through the ovariectomized female mouse model, the present disclosure finds that the 17β-estradiol/vitamin C molecular complex is distributed in a bone-targeted manner, and has an anti-endometrial hyperplasia effect and an anti-thrombosis effect.

The above experimental results confirm that the 17β-estradiol/vitamin C molecular complex composed of 17β-estradiol and vitamin C provided by the present disclosure is distributed to a bone tissue in a bone-targeted manner, which not only improves the anti-osteoporosis activity of 17β-estradiol but also eliminates the two lethal side effects of endometrial hyperplasia and thrombosis caused by 17β-estradiol.

### BRIEF DESCRIPTION OF THE DRAWINGS

FIG. 1 is an ESI (+)-FT-MS spectrum of a 17β-estradiol/vitamin C molecular complex in a molar ratio of 1:1.
FIG. 2 is an NOESY spectrum of a 17β-estradiol/vitamin C molecular complex in a molar ratio of 1:1.
FIG. 3 shows pathological sections of oviduct walls of ovariectomized mice treated with 17β-estradiol and a 17β-estradiol/vitamin C molecular complex in a molar ratio of 1:1,
   where (A) represents a pathological section of an oviduct wall with a thickness of 48.63 µm of a sham-operated mouse; (B) represents a pathological section of an oviduct wall with a thickness of 31.15 µm of an ovariectomized mouse treated with sodium carboxymethyl cellulose (CMC-Na); (C) represents a pathological section of an oviduct wall with a thickness of 152.11 µm of an ovariectomized mouse treated with 17β-estradiol at 2.3 µmol/kg/day; (D) represents a pathological section of an oviduct wall with a thickness of 60.20 µm of an ovariectomized mouse treated with a 17β-estradiol/vitamin C molecular complex in a molar ratio of 1:1 at 2.3 µmol/kg/day; and (E) represents a pathological section of an oviduct wall with a thickness of 46.88 µm of an ovariectomized mouse treated with a 17β-estradiol/vitamin C molecular complex in a molar ratio of 1:1 at 0.23 µmol/kg/day.

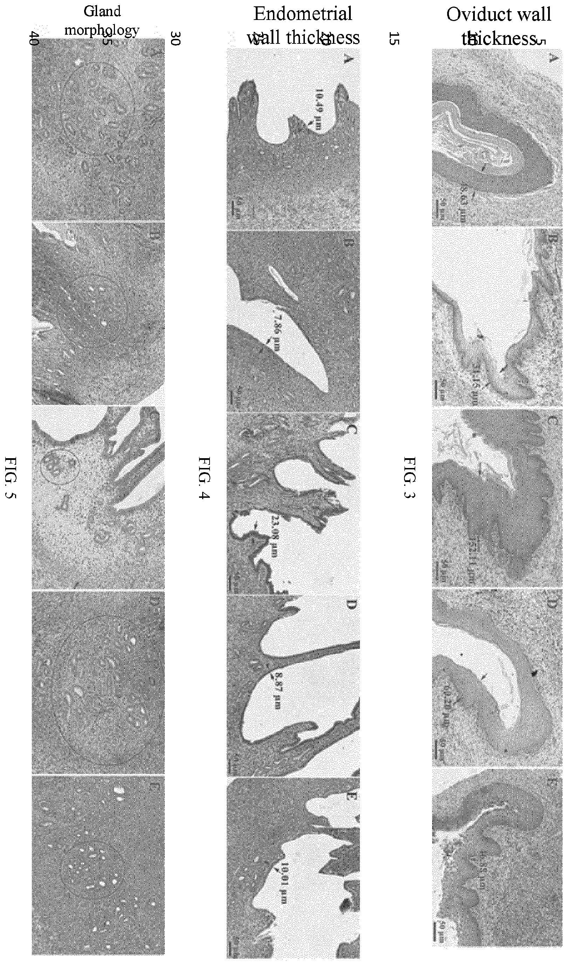

FIG. 4 shows pathological sections of endometria of ovariectomized mice treated with 17β-estradiol and a 17β-estradiol/vitamin C molecular complex in a molar ratio of 1:1,
   where (A) represents a pathological section of an endometrium with a thickness of 10.49 µm of a sham-operated mouse; (B) represents a pathological section of an endometrium with a thickness of 7.86 µm of an ovariectomized mouse treated with CMC-Na; (C) represents a pathological section of an endometrium with a thickness of 23.08 µm of an ovariectomized mouse treated with 17β-estradiol at 2.3 µmol/kg/day; (D) represents a pathological section of an endometrium with a thickness of 8.87 µm of an ovariectomized mouse treated with a 17β-estradiol/vitamin C molecular complex in a molar ratio of 1:1 at 2.3 µmol/kg/day; and (E) represents a pathological section of an endometrium with a thickness of 10.01 µm of an ovariectomized mouse treated with a 17β-estradiol/vitamin C molecular complex in a molar ratio of 1:1 at 0.23 µmol/kg/day.
FIG. 5 shows pathological sections of uterine glands of ovariectomized mice treated with 17β-estradiol and a 17β-estradiol/vitamin C molecular complex in a molar ratio of 1:1,
   where (A) represents a pathological section of a normal uterine gland of a sham-operated mouse; (B) represents a pathological section of a uterine gland without lesion of an ovariectomized mouse treated with CMC-Na; (C) represents a pathological section of a uterine gland of an ovariectomized mouse treated with 17β-estradiol at 2.3 µmol/kg/day, where the density of the tissue around the uterine gland is significantly reduced; (D) represents a pathological section of a uterine gland without lesion of an ovariectomized mouse treated with a 17β-estradiol/vitamin C molecular complex in a molar ratio of 1:1 at 2.3 µmol/kg/day; and (E) represents a pathological section of a uterine gland without lesion of an ovariectomized mouse treated with a 17β-estradiol/vitamin C molecular complex in a molar ratio of 1:1 at 0.23 µmol/kg/day.

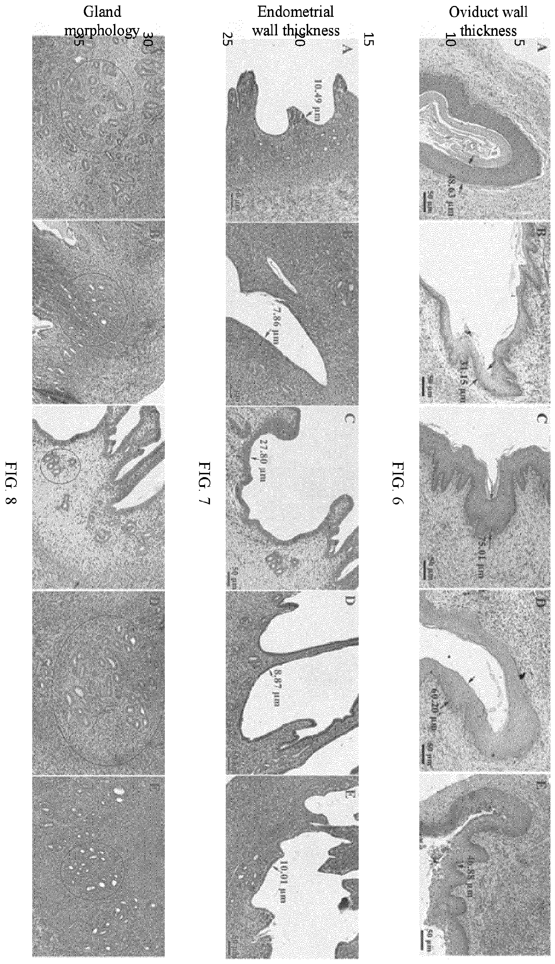

FIG. 6 shows pathological sections of oviduct walls of ovariectomized mice treated with Conjugated Estrogens Tablets and a 17β-estradiol/vitamin C molecular complex in a molar ratio of 1:1,
   where (A) represents a pathological section of an oviduct wall with a thickness of 48.63 µm of a sham-operated mouse; (B) represents a pathological section of an oviduct wall with a thickness of 31.15 µm of an ovariectomized mouse treated with CMC-Na; (C) represents a pathological section of an oviduct wall with a thickness of 75.01 µm of an ovariectomized mouse treated with Conjugated Estrogens Tablets at 0.104 mg/kg/day; (D) represents a pathological section of an oviduct wall with a thickness of 60.20 µm of an ovariectomized mouse treated with a 17β-estradiol/vitamin C molecular complex in a molar ratio of 1:1 at 2.3 µmol/kg/day; and (E) represents a pathological section of an oviduct wall with a thickness of 46.88 µm of an ovariectomized mouse treated with a 17β-estradiol/vitamin C molecular complex in a molar ratio of 1:1 at 0.23 µmol/kg/day.
FIG. 7 shows pathological sections of endometria of ovariectomized mice treated with Conjugated Estrogens Tablets and a 17β-estradiol/vitamin C molecular complex in a molar ratio of 1:1,
   where (A) represents a pathological section of an endometrium with a thickness of 10.49 µm of a sham-operated mouse; (B) represents a pathological section of an endometrium with a thickness of 7.86 µm of an ovariectomized mouse treated with CMC-Na; (C) represents a pathological section of an endometrium with a thickness of 27.80 µm of an ovariectomized mouse treated with Conjugated Estrogens Tablets at 0.104 mg/kg/day; (D) represents a pathological section of an endometrium with a thickness of 8.87 µm of an ovariectomized mouse treated with a 17β-estradiol/vitamin C molecular complex in a molar ratio of 1:1 at 2.3 µmol/kg/day; and (E) represents a pathological section of an endometrium with a thickness of 10.1 µm of an ovariectomized mouse treated with a 17β-estradiol/vitamin C molecular complex in a molar ratio of 1:1 at 0.23 µmol/kg/day.
FIG. 8 shows pathological sections of uterine glands of ovariectomized mice treated with Conjugated Estrogens Tablets and a 17β-estradiol/vitamin C molecular complex in a molar ratio of 1:1,
   where (A) represents a pathological section of a normal uterine gland of a sham-operated mouse; (B) represents a pathological section of a uterine gland without lesion of an ovariectomized mouse treated with CMC-Na; (C) represents a pathological section of a uterine gland of an ovariectomized mouse treated with Conjugated Estrogens Tablets at 0.104 mg/kg/day, where the density of a tissue around the uterine gland is significantly reduced; (D) represents a pathological section of a uterine gland without lesion of an ovariectomized mouse treated with a 17β-estradiol/vitamin C molecular complex in a molar ratio of 1:1 at 2.3 µmol/kg/day; and (E) represents a pathological section of a uterine gland without lesion of an ovariectomized mouse treated with a 17β-estradiol/vitamin C molecular complex in a molar ratio of 1:1 at 0.23 µmol/kg/day.

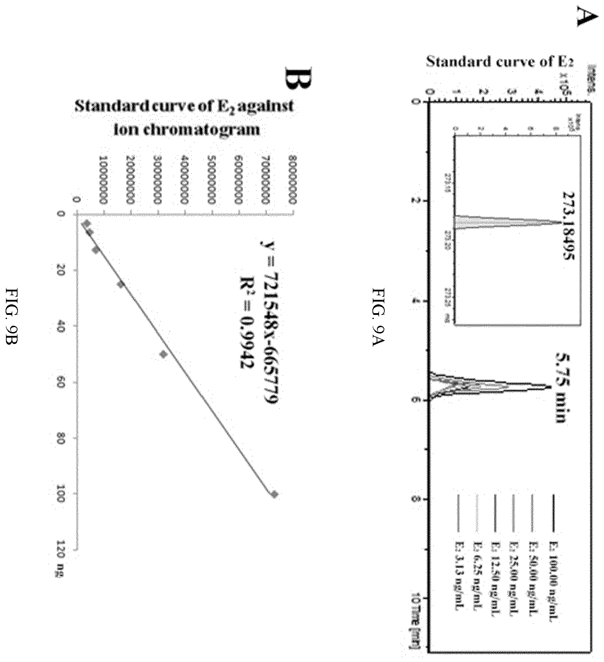

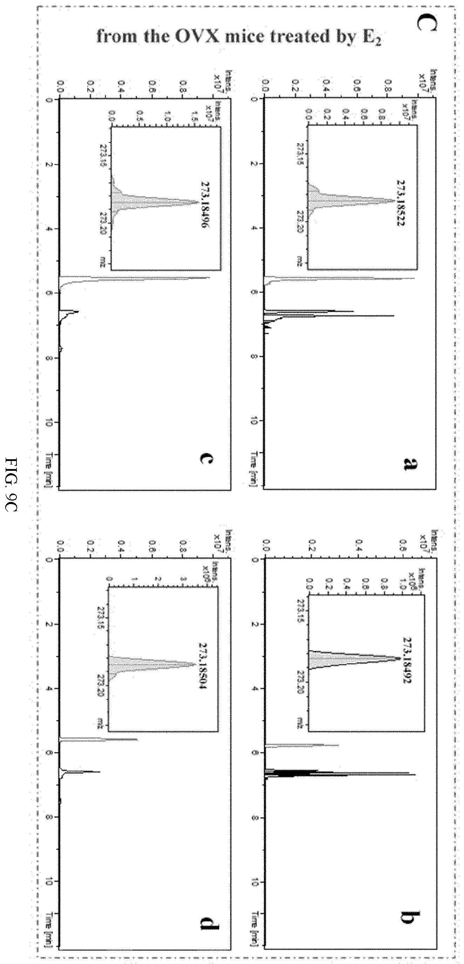

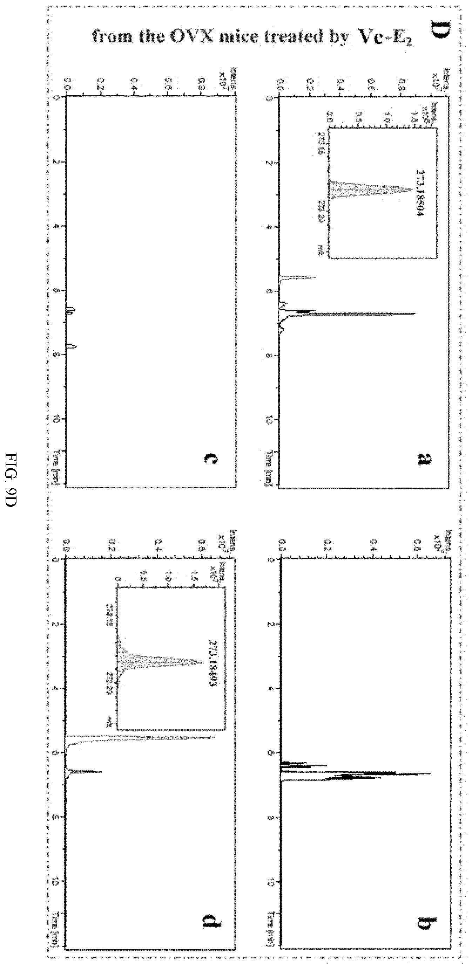

FIG. 9 shows a standard curve of 17β-estradiol and ion chromatograms of 17β-estradiol in organs of ovariectomized mice treated with 17β-estradiol (2.3 µmol/kg/day) and a 17β-estradiol/vitamin C molecular complex in a molar ratio of 1:1 (2.3 µmol/kg/day).
FIG. 9A shows an ion chromatogram of 17β-estradiol. FIG. 9B shows a standard curve of 17β-estradiol. FIG. 9C shows ion chromatograms of 17β-estradiol in organs of ovariectomized mice treated with 17β-estradiol (2.3 µmol/kg/day), where a, b, c, and d represent ion chromatograms of 17β-estradiol in the liver, blood, uterus, and femur, respectively. FIG. 9D shows ion chromatograms of 17β-estradiol in organs of ovariectomized mice treated with a 17β-estradiol/vitamin C molecular complex in a molar ratio of 1:1 (2.3 µmol/kg/day), where a, b, c, and d represent ion chromatograms of 17β-estradiol in the liver, blood, uterus, and femur, respectively.

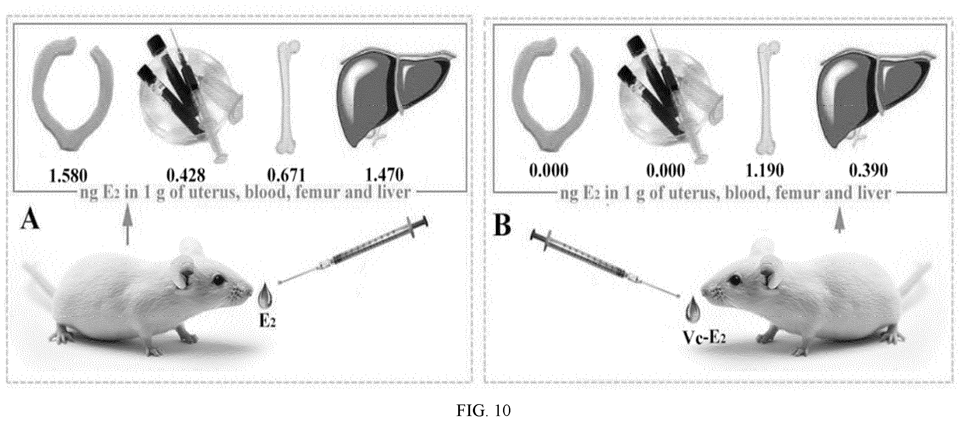

FIG. 10 shows 17β-estradiol contents in organs of ovariectomized mice treated with a 17β-estradiol/vitamin C molecular complex in a molar ratio of 1:1,
   where (A) shows 17β-estradiol contents in organs of ovariectomized mice treated with 17β-estradiol and (B) shows 17β-estradiol contents in organs of ovariectomized mice treated with the 17β-estradiol/vitamin C molecular complex.

### DETAILED DESCRIPTION OF THE EMBODIMENTS

In order to further illustrate the present disclosure, a series of examples are provided below. These examples are totally illustrative, which are provided merely to specifically describe the present disclosure and should not be construed as limiting the present disclosure.

### Example 1: Preparation of a 17β-estradiol/vitamin C molecular complex in a molar ratio of 1:1

2,720 mg of 17β-estradiol and 1,760 mg of vitamin C were dissolved in 60 mL of a 30% V/V aqueous ethanol solution to prepare a clear solution, and the clear solution was lyophilized to obtain 4,480 mg of a lyophilized powder.

### Example 2: Preparation of a 17β-estradiol/vitamin C molecular complex in a molar ratio of 1:1

2,720 mg of 17β-estradiol and 1,760 mg of vitamin C were mixed and dissolved in 50 mL of a 40% V/V aqueous ethanol solution to prepare a clear solution, and the clear solution was subjected to vacuum distillation at room temperature to obtain 4,480 mg of a powder.

### Example 3: Preparation of a 17β-estradiol/vitamin C molecular complex in a molar ratio of 1:1

2,720 mg of 17β-estradiol and 1,760 mg of vitamin C were mixed and dissolved in 40 mL of a 50% V/V aqueous ethanol solution to prepare a clear solution, and the clear solution was subjected to vacuum distillation at room temperature to obtain 4,480 mg of a powder.

### Example 4: Preparation of a 17β-estradiol/vitamin C molecular complex in a molar ratio of 1:1

2,720 mg of 17β-estradiol and 1,760 mg of vitamin C were mixed and dissolved in 35 mL of a 60% V/V aqueous ethanol solution to prepare a clear solution, and the clear solution was lyophilized to obtain 4,480 mg of a lyophilized powder.

### Example 5: Preparation of a 17β-estradiol/vitamin C molecular complex in a molar ratio of 0.25:1

680 mg of 17β-estradiol and 1,760 mg of vitamin C were mixed and dissolved in 35 mL of a 60% V/V aqueous ethanol solution to prepare a clear solution, and the clear solution was lyophilized to obtain 2,440 mg of a lyophilized powder.

### Example 6: Preparation of a 17β-estradiol/vitamin C molecular complex in a molar ratio of 0.5:1

1,360 mg of 17β-estradiol and 1,760 mg of vitamin C were mixed and dissolved in 50 mL of a 40% V/V aqueous ethanol solution to prepare a clear solution, and the clear solution was subjected to vacuum distillation at room temperature to obtain 3,120 mg of a powder.

### Example 7: Preparation of a 17β-estradiol/vitamin C molecular complex in a molar ratio of 0.75:1

2,040 mg of 17β-estradiol and 1,760 mg of vitamin C were mixed and dissolved in 40 mL of a 50% V/V aqueous ethanol solution to prepare a clear solution, and the clear solution was subjected to vacuum distillation at room temperature to obtain 3,880 mg of a powder.

### Example 8: Preparation of a 17β-estradiol/vitamin C molecular complex in a molar ratio of 1:0.25

2,720 mg of 17β-estradiol and 440 mg of vitamin C were mixed and dissolved in 35 mL of a 60% V/V aqueous ethanol solution to prepare a clear solution, and the clear solution was lyophilized to obtain 3,160 mg of a lyophilized powder.

### Example 9: Preparation of a 17β-estradiol/vitamin C molecular complex in a molar ratio of 1:0.5

2,720 mg of 17β-estradiol and 880 mg of vitamin C were dissolved in 60 mL of a 30% V/V aqueous ethanol solution to prepare a clear solution, and the clear solution was lyophilized to obtain 3,660 mg of a lyophilized powder.

### Example 10: Preparation of a 17β-estradiol/vitamin C molecular complex in a molar ratio of 1:0.75

2,720 mg of 17β-estradiol and 1,320 mg of vitamin C were mixed and dissolved in 50 mL of a 40% V/V aqueous ethanol solution to prepare a clear solution, and the clear solution was subjected to vacuum distillation at room temperature to obtain 4,040 mg of a powder.

### Experimental Example 1: Determination of an FT-MS spectrum of a 17β-estradiol/vitamin C molecular complex in a molar ratio of 1:1

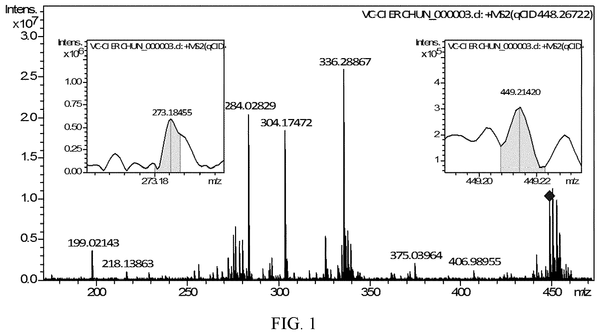

About 1 mg of the lyophilized powder of the 17β-estradiol/vitamin C molecular complex in a molar ratio of 1:1 prepared in Example 1 was dissolved in ultrapure water (UPW), and a resulting solution was tested for an ESI(+)-FT-MS spectrum. The ESI(+)-FT-MS spectrum in FIG. 1 shows a molecular ion with a mass number of 449.21420, which is equal to a mass number of one 17β-estradiol molecule: 272.17763 + a mass number of one vitamin C molecule: 176.03209 + 1 H. It can be seen that one 17β-estradiol molecule and one vitamin C molecule constitute the complex of the present disclosure. The qCID spectrum in FIG. 1 shows that, under ESI(+)-FT-MS determination conditions, the molecular ion with a mass number of 449.21420 is split into an estradiol ion with a mass number of 273.18455 + 1 H and a vitamin C ion with a mass number of 199.02143 + 1 Na. It can be seen that the 17β-estradiol/vitamin C molecular complex in a molar ratio of 1:1 is the only form in which 17β-estradiol and vitamin C exist.

### Experimental Example 2: Determination of an NOESY spectrum of a 17β-estradiol/vitamin C molecular complex in a molar ratio of 1:1

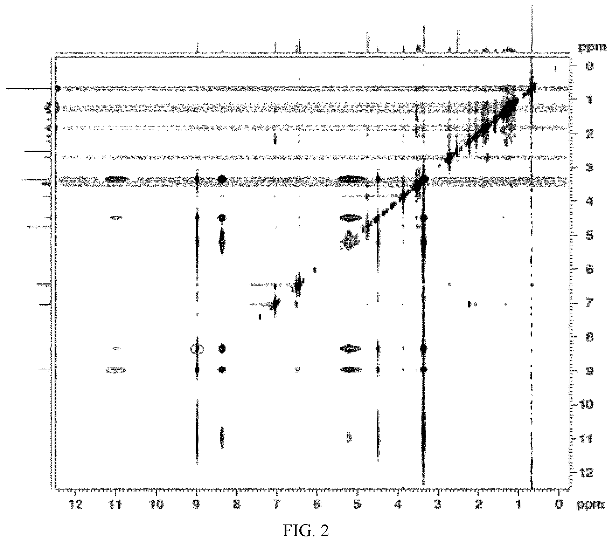

About 5 mg of the lyophilized powder of the 17β-estradiol/vitamin C molecular complex in a molar ratio of 1:1 prepared in Example 1 was dissolved in deuterated dimethylsulfoxide (DMSO), and a resulting solution was tested on an 800 M nuclear magnetic resonance (NMR) apparatus for an NOESY spectrum. The result is shown in FIG. 2. FIG. 2 shows that the complex has two important correlated peaks in the NOESY spectrum. The peaks appear due to the interaction between an aromatic proton in 17β-estradiol and an enol proton in vitamin C, that is, a distance between the protons is less than 4 Å. Thus, 17β-estradiol and vitamin C approach each other according to such a requirement to form the molecular complex.

### Experimental Example 3: Evaluation of the influence of the 17β-estradiol/vitamin C molecular complex on the trabecular bone density in ovariectomized female mice

The trabecular bone density in ovariectomized female mice is an important index for anti-osteoporosis activity. In order to evaluate the anti-osteoporosis activity of the 17β-estradiol/vitamin C molecular complex (prepared in Example 1), the present disclosure first evaluated the influence of the 17β-estradiol/vitamin C molecular complex on the trabecular bone density in ovariectomized female mice. ICR mice (each with a weight of 25.0 ± 2.0 g) were subjected to oophorectomy. Day 7 after the oophorectomy, a sham-operated group (Sham group), an oophorectomy + CMC-Na group, an oophorectomy + estradiol group (at an oral dose of 2.3 µmol/kg), and oophorectomy + 17β-estradiol/vitamin C molecular complex (prepared in Example 1) groups (at oral doses of 2.3 µmol/kg, 0.23 µmol/kg, and 0.023 µmol/kg) were set with 11 mice in each group. The oral administration started on the day of grouping, and the mice in each group were continuously administered for 4 weeks. On the day after the last administration, the mice were weighed, blood was collected from the orbit, and then the mice were anesthetized with diethyl ether and sacrificed. A left femur was collected from the mice, a muscle tissue attached thereto was completely removed, the left femur was soaked in chloroform/methanol (2/1) twice (3 h each time) for degreasing, and the trabecular bone density at a position 0.2 mm to 0.4 mm below a femoral articular head was determined on a computed tomography (CT) scanner. The results in Table 1 show that the 17β-estradiol/vitamin C molecular complex of the present disclosure can inhibit the decrease in the trabecular bone density in ovariectomized mice in a dose-dependent manner. A minimum effective dose (MED) of the 17β-estradiol/vitamin C molecular complex was 0.023 µmol/kg. At a dose of 0.23 µmol/kg, 17β-estradiol exhibited no therapeutic effect.

**Table 1 Trabecular bone densities in ovariectomized mice administered orally with the complex**

| Group | Dose (µmol/kg/day) | Trabecular bone density (mean ± SD mg/cm³) |
|---|---|---|
| Sham-operated group | -- | 758.56 ± 66.41 |
| Oophorectomy + CMC-Na group | -- | 586.08 ± 26.11 |
| Oophorectomy + conjugated estrogens group | 104 | 750.94 ± 35.55^{a} |
| Oophorectomy + estradiol group | 2.3 | 748.57 ± 33.37^{a} |
| | 0.23 | 595.35 ± 30.47^{d} |
| Oophorectomy + 17β-estradiol/vitamin C molecular complex groups | 2.3 | 747.49 ± 40.56^{b} |
| | 0.23 | 651.88 ± 26.06^{c} |
| | 0.023 | 606.63 ± 41.90^{d} |

| | | |
|---|---|---|
| a) means that P < 0.01 compared with mice in the oophorectomy + CMC-Na group and , P > 0.05 compared with mice in the sham-operated group and the oophorectomy + 2.3 µmol/kg/day estradiol group; b) means that P < 0.05 compared with mice in the oophorectomy + CMC-Na group, P *<* 0.01 compared with mice in the oophorectomy + 2.3 µmol/kg/day complex group, and P *<* 0.05 compared with mice in the oophorectomy + 0.023 µmol/kg/day complex group; c) means that P < 0.01 compared with mice in the oophorectomy + CMC-Na group, the oophorectomy + 2.3 µmol/kg/day complex group, and the oophorectomy + 0.023 µmol/kg/day complex group; and d) means that P > 0.05 compared with mice in the oophorectomy + CMC-Na group; and n = 11. | | |

### Experimental Example 4: Evaluation of the influence of the 17β-estradiol/vitamin C molecular complex on the femoral dry weight in ovariectomized female mice

The femoral dry weight in ovariectomized female mice is one of the important indexes for anti-osteoporosis activity. In order to evaluate the anti-osteoporosis activity of the 17β-estradiol/vitamin C molecular complex (prepared in Example 1), the present disclosure evaluated the influence of the 17β-estradiol/vitamin C molecular complex on the femoral dry weight in ovariectomized female mice. In Experimental Example 3, the left femur of each of the mice whose trabecular bone density was determined was dried in an oven at 120°C for 6 h, and then a femoral dry weight was determined. Results are shown in Table 2. The results showed that the 17β-estradiol/vitamin C molecular complex could effectively inhibit the femoral weight loss in ovariectomized mice at the three doses. An MED of the 17β-estradiol/vitamin C molecular complex was 0.023 µmol/kg. Even at a dose of 0.23 µmol/kg, 17β-estradiol exhibited no therapeutic effect.

**Table 2 Femoral dry weights in ovariectomized mice administered orally with the complex**

| Group | Dose (µmol/kg/day) | Femoral dry weight (mean ± SD mg) |
|---|---|---|
| Sham-operated group | -- | 52.42±3.62 |
| Oophorectomy + CMC-Na group | -- | 47.35±5.24 |
| Oophorectomy + conjugated estrogens group | 104 | 51.00 ± 2.34a |
| Oophorectomy + 17β-estradiol group | 2.3 | 54.42±2.96^{a} |
| | 0.23 | 49.08 ± 6.02^{b} |
| Oophorectomy + 17β-estradiol/vitamin C molecular complex groups | 2.3 | 54.16±4.71^{a} |
| | 0.23 | 53.54±4.59^{a} |
| | 0.023 | 54.70±2.29^{a} |

| | | |
|---|---|---|
| a) means that P < 0.01 compared with mice in the CMC-Na group and P > 0.05 compared with mice in the sham-operated group; b) means that P > 0.05 compared with mice in the CMC-Na group; and n = 11. | | |

### Experimental Example 5: Evaluation of the influence of the 17β-estradiol/vitamin C molecular complex on the femoral ash weight in ovariectomized female mice

An ash weight of a calcined femur in ovariectomized female mice is one of the important indexes for anti-osteoporosis activity. In order to evaluate the anti-osteoporosis activity of the 17β-estradiol/vitamin C molecular complex, the present disclosure evaluated the influence of the molecular complex on an ash weight of a calcined femur in ovariectomized female mice. In Experimental Example 3, the left femur of each of the mice whose trabecular bone density was determined was dried in an oven at 120°C for 6 h and then calcined in a muffle furnace at 800°C for 8 h, and then an ash weight of the femur was determined. Results are shown in Table 3. The results showed that the 17β-estradiol/vitamin C molecular complex could effectively inhibit the femoral ash weight loss in ovariectomized mice at the three doses. An MED of the 17β-estradiol/vitamin C molecular complex was 0.023 µmol/kg. Even at a dose of 0.23 µmol/kg/day, 17β-estradiol exhibited no therapeutic effect.

**Table 3 Femoral ash weights in ovariectomized mice administered orally with the complex**

| Group | Dose (µmol/kg/day) | Femoral ash weight (mean ± SD mg) |
|---|---|---|
| Sham-operated group | -- | 32.53±1.83 |
| Oophorectomy + CMC-Na group | -- | 26.91±4.10 |
| Oophorectomy + conjugated estrogens group | 104 | 31.07 ± 1.44^{a} |
| Oophorectomy + estradiol group | 2.3 | 33.37±3.21^{a} |
| | 0.23 | 29.08 ± 5.42^{b} |
| Oophorectomy + 17β-estradiol/vitamin C molecular complex groups | 2.3 | 32.89±3.17^{a} |
| | 0.23 | 31.58±3.60^{a} |
| | 0.023 | 30.75±1.79^{a} |

| | | |
|---|---|---|
| a) means that P < 0.01 compared with mice in the CMC-Na group and P > 0.05 compared with mice in the sham-operated group; b) means that P > 0.05 compared with mice in the CMC-Na group; and n = 11. | | |

### Experimental Example 6: Evaluation of the influence of the 17β-estradiol/vitamin C molecular complex on the femoral mineral content in ovariectomized female mice

The femoral mineral content (BMC) in ovariectomized female mice is an important index for anti-osteoporosis activity. The femoral mineral content is defined as a ratio of a femoral ash weight to a femoral dry weight. In order to evaluate the anti-osteoporosis activity of the 17β-estradiol/vitamin C molecular complex, the present disclosure evaluated the influence of the molecular complex on the ratio of a femoral ash weight to a femoral dry weight in ovariectomized female mice. The ash weight of the left femur of each of the mice determined in Experimental Example 5 was divided by the dry weight of the left femur of each of the mice determined in Experimental Example 4 to obtain a ratio of the femoral ash weight to the femoral dry weight, which was the femoral mineral content. Results are shown in Table 4. The results indicated that the 17β-estradiol/vitamin C molecular complex inhibited the decrease in the femoral mineral content of ovariectomized mice in a dose-dependent manner. An MED of the 17β-estradiol/vitamin C molecular complex was 0.023 µmol/kg. Even at a dose of 0.23 µmol/kg/day, 17β-estradiol exhibited no therapeutic effect.

**Table 4 Femoral mineral contents in ovariectomized mice administered orally with the complex**

| Group | Dose (µmol/kg/day) | BMC (mean ± SD mg/mg) |
|---|---|---|
| Sham-operated group | -- | 0.632 ±0.030 |
| Oophorectomy + CMC-Na group | -- | 0.569 ±0.037 |
| Oophorectomy + conjugated estrogens group | 104 | 0.609 ±0.018^{a} |
| Oophorectomy + estradiol group | 2.3 | 0.620±0.020^{a} |
| | 0.23 | 0.560 ± 0.041 |
| Oophorectomy + 17β-estradiol/vitamin C molecular complex groups | 2.3 | 0.629±0.034^{b} |
| | 0.23 | 0.601±0.024^{c} |
| | 0.023 | 0.577±0.023^{d} |
| | | |

| | | |
|---|---|---|
| a) means that P < 0.01 compared with mice in the CMC-Na group and P > 0.05 compared with mice in the sham-operated group; b) means that P < 0.01 compared with mice in the CMC-Na group and P > 0.05 compared with mice in the sham-operated group and the oophorectomy + 2.3 µmol/kg/day estradiol group; c) means that P < 0.05 compared with mice in the CMC-Na group, p < 0.05 compared with mice in the estradiol group, and p < 0.05 compared with mice in the oophorectomy + 2.3 µmol/kg/day 17β-estradiol/vitamin C molecular complex group and the oophorectomy + 0.023 µmol/kg/day 17β-estradiol/vitamin C molecular complex; d) means that P > 0.05 compared with mice in the CMC-Na group; and n = 11. | | |

### Experimental Example 7: Evaluation of the influence of the 17β-estradiol/vitamin C molecular complex on a ratio of a femoral ash weight to a femoral length in ovariectomized female mice

In order to clarify whether the effective inhibition of the 17β-estradiol/vitamin C molecular complex on the femoral ash weight loss in ovariectomized mice at the three doses is related to the growth of mouse femurs, the present disclosure measured a femoral length and calculated a ratio of the femoral ash weight to the femoral length. Results are shown in Table 5. The results showed that the 17β-estradiol/vitamin C molecular complex could effectively inhibit the decrease in the ratio of the femoral ash weight to the femoral length in ovariectomized mice but had no impact on the femoral length. It can be seen that the 17β-estradiol/vitamin C molecular complex had no impact on the growth of femurs, and its effective inhibition on the decrease in the ratio of the femoral ash weight to the femoral length in ovariectomized mice was a result of its effective inhibition on the femoral mineral loss. An MED of the molecular complex was 0.023 µmol/kg. Even at a dose of 0.23 µmol/kg/day, 17β-estradiol exhibited no therapeutic effect.

**Table 5 Ratios of the femoral ash weight to the femoral length in ovariectomized mice administered orally with the complex**

| Group | Dose (µmol/kg/day) | Femoral length (mean ± SD mm) | Ash weight/length (mean ± SD mg/mm) |
|---|---|---|---|
| Sham-operated group | -- | 16.74 ±0.98 | 1.95 ±0.11 |
| Oophorectomy + CMC-Na group | -- | 16.98 ±0.40 | 1.62 ±0.27 |
| Oophorectomy + conjugated estrogens group | 104 | 16.37 ±0.30 | 1.90 ±0.08a |
| Oophorectomy + estradiol group | 2.3 | 16.72 ±0.35 | 2.02 ±0.20^{a} |
| | 0.23 | 16.87 ±0.34 | 1.72 ±0.22 |
| Oophorectomy + 17β-estradiol/vitamin C molecular complex groups | 2.3 | 17.02 ±0.48 | 1.99 ±0.17^{a} |
| | 0.23 | 17.13 ±0.34 | 1.86 ±0.16^{a} |
| | 0.023 | 16.69 ±0.29 | 1.87 ±0.09^{a} |

| | | | |
|---|---|---|---|
| a) means that P< 0.01 compared with mice in the CMC-Na group; and n = 11. | | | |

### Experimental Example 8: Evaluation of the influence of the 17β-estradiol/vitamin C molecular complex on the femoral calcium content in ovariectomized female mice

The femoral calcium content in ovariectomized female mice is an important index for anti-osteoporosis activity. In order to evaluate the anti-osteoporosis activity of the 17β-estradiol/vitamin C molecular complex, the present disclosure evaluated the influence of the 17β-estradiol/vitamin C molecular complex on a femoral calcium content in ovariectomized female mice. The ovariectomized mice were treated continuously for 4 weeks. The day after the last administration, blood was collected from the orbit, allowed to stand for 30 min, and centrifuged at 3,000 g/min for 20 min to obtain serum. Then a femoral calcium content was determined by the o-cresolphthalein complexone (OCPC) method. As shown in Table 6, the 17β-estradiol/vitamin C molecular complex could effectively inhibit the decrease in the femoral calcium content in ovariectomized female mice at doses of 2.3 µmol/kg and 0.23 µmol/kg, and an MED was 0.23 µmol/kg/day. At a dose of 0.23 µmol/kg/day, 17β-estradiol itself exhibited no therapeutic effect.

**Table 6 Femoral calcium content in ovariectomized mice administered orally with the molecular complex**

| Group | Dose (µmol/kg/day) | Femoral calcium content (mean ± SD %) |
|---|---|---|
| Sham-operated group | -- | 24.53±1.03 |
| Oophorectomy + CMC-Na group | -- | 23.18±0.79 |
| Oophorectomy + conjugated estrogens group | 104 | 24.42 ±1.80^{a} |
| Oophorectomy + estradiol group | 2.3 | 24.57±1.85^{a} |
| | 0.23 | 23.34 ±0.70 |
| Oophorectomy + 17β-estradiol/vitamin C molecular | 2.3 | 25.12±2.21^{a} |
| | 0.23 | 24.86±0.92^{a} |
| complex groups | 0.023 | 23.29±2.11 |

| | | |
|---|---|---|
| a) means that P < 0.01 compared with mice in the CMC-Na group and P > 0.05 compared with mice in the sham-operated group; and n = 11. | | |

### Experimental Example 9: Evaluation of the side effect of endometrial hyperplasia in the treatment of ovariectomized female mice with the 17β-estradiol/vitamin C molecular complex

Endometrial hyperplasia is one of the major side effects of 17β-estradiol therapy. In order to investigate whether the treatment with the 17β-estradiol/vitamin C molecular complex (prepared in Example 1) can prevent this side effect, the present disclosure evaluated the influence of the 17β-estradiol/vitamin C molecular complex on the uterine weight of ovariectomized female mice. The data in Table 7 showed that the 17β-estradiol significantly increased the uterine weight of ovariectomized female mice at both doses of 2.3 µmol/kg/day and 0.23 µmol/kg/day, indicating that the 17β-estradiol induced endometrial hyperplasia. The 17β-estradiol/vitamin C molecular complex at any of the three doses of 2.3 µmol/kg/day, 0.23 µmol/kg/day, and 0.023 µmol/kg/day did not cause a uterine weight gain in ovariectomized female mice, indicating that the 17β-estradiol/vitamin C molecular complex did not induce endometrial hyperplasia. The results in Table 7 also showed that, although the CMC-Na-treated ovariectomized mice exhibited the expected uterine atrophy, the 17β-estradiol/vitamin C molecular complex did not induce uterine atrophy in ovariectomized female mice even at a dose as low as 0.023 µmol/kg/day. That is, the treatment with the 17β-estradiol/vitamin C molecular complex does not cause both endometrial hyperplasia and uterine atrophy.

**Table 7 Uterine weights in ovariectomized mice administered orally with the complex**

| Group | Dose (µmol/kg) | Uterine weight (mean ± SD g) |
|---|---|---|
| Sham-operated group | -- | 0.111 ± 0.033 |
| Oophorectomy + CMC-Na group | -- | 0.061 ± 0.021 |
| Oophorectomy + estradiol group | 2.3 | 0.207 ± 0.045^{a} |
| | 0.23 | 0.200 ± 0.041^{a} |
| Oophorectomy + 17β-estradiol/vitamin C molecular complex groups | 2.3 | 0.115 ± 0.028^{b} |
| | 0.23 | 0.101 ± 0.024^{b} |
| | 0.023 | ± 0.026^{c} |

| | | |
|---|---|---|
| a) means that P < 0.01 compared with mice in the CMC-Na group and the sham-operated group; b) *P* > 0.05 compared with mice in the sham-operated group; c) means that P < 0.05 compared with mice in the CMC-Na group and the sham-operated group; and n = 11. | | |

Pathological sections of oviduct walls, endometria, and uterine glands of ovariectomized female mice were prepared to evaluate the influence of the complex on the oviduct walls, endometria, and uterine glands of the ovariectomized mice.

Results are shown in FIG. 3 to FIG. 8. These pathological sections showed that, although the 17β-estradiol and Conjugated Estrogens Tablets could induce the thickening of endometria and oviduct walls in mice, the 17β-estradiol/vitamin C molecular complex in a molar ratio of 1:1 did not exhibit this side effect. These pathological sections also showed that, although the 17β-estradiol and Conjugated Estrogens Tablets could cause lesions of uterine glands in mice, the 17β-estradiol/vitamin C molecular complex in a molar ratio of 1:1 did not exhibit this side effect.

### Example 14: Evaluation of the side effect of thrombosis in the treatment of ovariectomized female mice with the 17β-estradiol/vitamin C molecular complex

Thrombosis is another major side effect of 17β-estradiol therapy. In order to investigate whether the treatment with the 17β-estradiol/vitamin C molecular complex (prepared in Example 1) can prevent this side effect, the present disclosure evaluated a tail bleeding time in ovariectomized female mice treated with the 17β-estradiol/vitamin C molecular complex. 30 min after the last administration, each of the mice was fixed in a fixing device with a tail exposed, the tail was quickly cut at 2 mm from the tip of the tail such that an incision bled naturally, and the timing was started. Blood drops from the incision were gently wiped away with filter paper every 30 s until the bleeding was spontaneously stopped. A time from the start of bleeding to the stop of bleeding is the bleeding time. The results in Table 8 showed that the 17β-estradiol significantly shortened the tail bleeding time in ovariectomized female mice at both doses of 2.3 µmol/kg/day and 0.23 µmol/kg/day. However, the 17β-estradiol/vitamin C molecular complex at any of the three doses of 2.3 µmol/kg/day, 0.23 µmol/kg/day, and 0.023 µmol/kg/day did not affect the tail bleeding time in ovariectomized female mice. It can be seen that the 17β-estradiol/vitamin C molecular complex overcame the thrombosis risk of 17β-estradiol treatment.

**Table 8 Tail bleeding time in ovariectomized mice administered orally with the complex**

| Group | Dose (µmol/kg/day) | Bleeding time (mean ± SD s) |
|---|---|---|
| Sham-operated group | -- | 858.4 ± 163.7 |
| Oophorectomy + CMC-Na group | -- | 853.4 ± 159.6 |
| Oophorectomy + conjugated estrogens group | 104 | 679.7 ± 244.3a |
| Oophorectomy + estradiol group | 2.3 | 659.1 ± 203.8^{a} |
| | 0.23 | 640.6 ± 189.6^{a} |
| Oophorectomy + 17β-estradiol/vitamin C molecular complex groups | 2.3 | 824.3 ± 157.7^{b} |
| | 0.23 | 817.1 ± 153.9^{b} |
| | 0.023 | 847.7± 148.9^{b} |

| | | |
|---|---|---|
| a) means that P < 0.05 compared with mice in the CMC-Na group and the sham-operated group; b) means that P > 0.05 compared with mice in the CMC-Na group and the sham-operated group; and n = 11. | | |

### Experimental Example 10: Evaluation of the distribution of the 17β-estradiol/vitamin C molecular complex in organs of ovariectomized female mice

In order to understand the principle of the 17β-estradiol/vitamin C molecular complex (prepared in Example 1) to exhibit high anti-osteoporosis activity and completely prevent the side effects of 17β-estradiol in the treatment of ovariectomized female mice, the present disclosure used the HPLC-FT-MS ion chromatography to quantitatively determine the accumulation of 17β-estradiol in the liver, blood, uterus, bone, brain, spleen, and kidney of treated ovariectomized female mice. A standard curve of 17β-estradiol and ion chromatograms of 17β-estradiol in organs of ovariectomized mice treated with 17β-estradiol (2.3 µmol/kg/day) and a 17β-estradiol/vitamin C molecular complex in a molar ratio of 1:1 µmol/kg/day) were shown in FIG. 9.

For this reason, a linear equation was established for an area relationship between 17β-estradiol standard solutions with reaction concentrations of 3.13 ng/mL, 6.25 ng/mL, 12.50 ng/mL, 25.0 ng/mL, 50.00 ng/mL, and 100.00 ng/mL and ion currents (y = 721548x - 665779, R² = 0.9942). This linear equation was used to quantify the accumulation of 17β-estradiol in the liver, blood, uterus, bone, brain, spleen, and kidney of ovariectomized female mice. The results showed that 17β-estradiol was not detected in the brain, spleen, and kidney of ovariectomized female mice treated with 17β-estradiol. Amounts of 17β-estradiol in 1 g of liver, 1 g of blood, 1 g of uterus, and 1 g of bone of ovariectomized female mice treated with 17β-estradiol were 1.470 ng, 0.428 ng, 1.580 ng, and 0.671 ng, respectively, as shown in FIG. 9C. Amounts of 17β-estradiol in the organs of ovariectomized mice treated with 17β-estradiol were shown in FIG. 10. Since 17β-estradiol accumulates in a large amount in the liver of the ovariectomized female mice treated thereby, the first pass metabolism results in a low activity. Since 17β-estradiol accumulates in a large amount in the uterus of the ovariectomized female mice treated thereby, the side effect of uterine weight gain is strong. Since 17β-estradiol accumulates significantly in the blood of the ovariectomized female mice treated thereby, there is a risk of thrombosis. The linear equation was used to quantify the accumulation of 17β-estradiol in the liver, blood, uterus, bone, brain, spleen, and kidney of ovariectomized female mice treated by the 17β-estradiol/vitamin C molecular complex. The results showed that 17β-estradiol was not detected in the blood, uterus, brain, spleen, and kidney of ovariectomized female mice treated with the 17β-estradiol/vitamin C molecular complex, as shown in FIG. 9D. 17β-estradiol was detected in the liver and femur, and amounts of 17β-estradiol in 1 g of liver and 1 g of bone of ovariectomized female mice treated with the 17β-estradiol/vitamin C molecular complex were 0.390 ng and 1.190 ng, respectively (FIG. 10). It can be seen that the treatment with the 17β-estradiol/vitamin C molecular complex could make 17β-estradiol clearly distributed in a bone-target manner.

It should be stated that the content and specific implementations of the present disclosure are intended to illustrate the practical application of the technical solutions provided by the present disclosure, rather than to limit the protection scope of the present disclosure. Those skilled in the art can make various modifications, equivalent substitutions, or improvements within the spirit and principle of the present disclosure.

## Claims

1. A 17β-estradiol/vitamin C molecular complex, wherein the molecular complex is obtained by compounding 17β-estradiol with vitamin C, wherein a distance between an aromatic proton in the 17β-estradiol and an enol proton in the vitamin C is less than 4 Å.

2. The 17β-estradiol/vitamin C molecular complex according to claim 1, wherein the molecular complex is obtained by compounding the 17β-estradiol with the vitamin C in a molar ratio of 0.25:1, 0.5:1, 0.75:1, 1:1, 1:0.25, 1:0.5, or 1:0.75.

3. The 17β-estradiol/vitamin C molecular complex according to claim 2, wherein the molecular complex is obtained by compounding the 17β-estradiol with the vitamin C in a molar ratio of 1:1.

4. The 17β-estradiol/vitamin C molecular complex according to claim 3, wherein a mass number of molecular ion of the molecular complex in an ESI(+)-FT-MS spectrum is 449.21420, wherein the mass number of molecular ion of the molecular complex in the ESI(+)-FT-MS spectrum is equal to a sum of a mass number of one 17β-estradiol molecule, a mass number of one vitamin C molecule, and 1 H, wherein the mass number of one 17β-estradiol molecule is 272.17763 and the mass number of one vitamin C molecule is 176.03209.

5. The 17β-estradiol/vitamin C molecular complex according to claim 3, wherein in a qCID spectrum of the molecular complex under ESI(+)-FT-MS conditions, the molecular ion with a mass number of 449.21420 is split into a 17β-estradiol ion with a mass number of 273.18455 + 1 H and a vitamin C ion with a mass number of 199.02143 + 1 Na, that is, a 17β-estradiol/vitamin C molecular complex in a molar ratio of 1:1 is the only form in which 17β-estradiol and vitamin C exist.

6. A preparation method of the 17β-estradiol/vitamin C molecular complex according to any one of claims 1 to 4, comprising the following steps:
1) mixing the 17β-estradiol and the vitamin C in a molar ratio of 0.25:1, 0.5:1, 0.75:1, 1:1, 1:0.25, 1:0.5, or 1:0.75 to obtain a mixture;
2) dissolving the mixture in an aqueous ethanol solution to prepare a clear solution; and
3) subjecting the clear solution to solvent removal to obtain a solid powder, wherein the solid powder is the 17β-estradiol/vitamin C molecular complex.

7. The preparation method according to claim 6, wherein in step 1), a molar ratio of the 17β-estradiol to the vitamin C is 1:1; and in step 2), based on V/V, the aqueous ethanol solution is a 30% to 60% aqueous ethanol solution.

8. The preparation method according to claim 6, wherein in step 3), the solvent removal is a process by any one of lyophilization, vacuum distillation, and spray drying; and preferably, the solvent removal is a process by lyophilization.

9. A use of the 17β-estradiol/vitamin C molecular complex according to any one of claims 1 to 5 in the preparation of an anti-osteoporosis drug.

10. A use of the 17β-estradiol/vitamin C molecular complex according to any one of claims 1 to 5 in the preparation of a drug for inhibiting endometrial hyperplasia.
